# EUROPÄISCHE PATENTANMELDUNG

(11) **EP 3 053 615 A1**
(43) Veröffentlichungstag der Anmeldung: **10.08.2016**
(21) Anmeldenummer: 15154080.4
(22) Anmeldetag: 06.02.2015
(51) Int. Cl.: A61M 1/36

(54) **Verfahren zum Abscheiden von Gasblasen aus einer Kardioplegielösung und Blasenabscheider**

(71) Anmelder: Sefar AG, 9410 Heiden (CH)
(72) Erfinder: Gerdes, Gerd, 9030 Abtwil (CH); Weber, Jérémie, 6833 Klaus (AT); Zäch, Andreas, 9463 Oberriet (CH); Dür, Hansjörg, 6858 Schwarzach (AT)
(74) Vertreter: Wunderlich, Rainer

(57) **Zusammenfassung**

Die Erfindung betrifft ein Verfahren zum Abscheiden von Gasblasen aus einer Kardioplegielösung mit einem Blasenabscheider (10), wobei die Kardioplegielösung über einen Zulauf (14) in ein Gehäuse (12) mit einem Filterelement (30) geleitet wird, die Kardioplegielösung das Filterelement (30) durchströmt, wobei Gasblasen in der Kardioplegielösung an dem Filterelement (30) rückgehalten werden, und die zurückgehaltenen Gasblasen an dem Filterelement (30) nach oben zu einer Auffangkammer (40) abgeführt werden. Gemäß der Erfindung ist vorgesehen, dass als Filterelement (30) als eine dünnwandige Hülse (31) mit einem Filtermaterial verwendet wird, welches mit einer Poren- oder Maschenweite gebildet ist, welche kleiner als 50 µm ist.

## Beschreibung

Die Erfindung betrifft ein Verfahren zum Abscheiden von Gasblasen aus einer Kardioplegielösung mit einem Blasenabscheider, wobei die Kardioplegielösung über einen Zulauf in ein Gehäuse mit einem Filterelement geleitet wird, die Kardioplegielösung das Filterelement durchströmt, wobei Gasblasen in der Kardioplegielösung an dem Filterelement zurückgehalten werden und die zurückgehaltenen Gasblasen an dem Filterelement nach oben zu einer Auffangkammer abgeführt werden, gemäß dem Oberbegriff des Anspruchs 1.

Die Erfindung betrifft weiterhin einen Blasenabscheider zum Abscheiden von Gasblasen aus einer Flüssigkeit, insbesondere einer Kardioplegielösung, mit einem Gehäuse, welches einen Zulauf und einen Ablauf für die Flüssigkeit aufweist, einen in dem Gehäuse angeordneten Filterelement, welches von der Flüssigkeit durchströmbar ist, wobei Gasblasen in der Flüssigkeit an dem Filterelement zurückgehalten werden und eine Auffangkammer, welche oberhalb des Filterelementes in dem Gehäuse angeordnet und zum Auffangen der zurückgehaltenen Gasblasen ausgebildet ist, gemäß dem Oberbegriff des Anspruchs 4.

Kardioplegielösungen werden bei Herzoperationen eingesetzt, bei denen es erforderlich ist, ein Schlagen des Herzens zu unterbinden. Zu diesem Zweck wird ein Patient an einer Herz-Lungen-Maschine angeschlossen, um einen lebensnotwendigen Gasaustausch im Blutkreislauf des Patienten sicherzustellen. Dabei wird dem Blut einerseits CO₂ entzogen und andererseits dieses mit Sauerstoff angereichert.

Bei der Zuführung einer Kardioplegielösung ist dafür Sorge zu tragen, dass keine oder zumindest keine übermäßig großen Gasblasen in den Blutkreislauf des Patienten eingebracht werden. Dies könnte zu schwerwiegenden Folgen für den Patienten führen.

Bei der Zuführung einer Kardioplegielösung von außerhalb des Körpers in den Blutkreislauf ist es notwendig und üblich, in der Zuführungsleitung einen sogenannten Blasenabscheider vorzusehen. Dieser umfasst ein Filterelement, welches Gasblasen ab einer bestimmten Größe zurückhalten kann. Als Filterelemente können dabei Membrane oder andere sehr feine Trennmedien eingesetzt werden. Diese können einen sehr hohen Durchflusswiderstand aufweisen, was zu einem hohen Differenzdruck führt. Bei herkömmlichen Blasenabscheidern für Kardioplegielösungen ist es gebräuchlich, diese mit einem Wärmetauscher zu kombinieren, um eine gewünschte Temperatur einzustellen oder zu halten.

Der Erfindung liegt die Aufgabe zugrunde, ein Verfahren zum Abscheiden von Gasblasen aus einer Kardioplegielösung und einen Blasenabscheider anzugeben, mit welchen bei einem kompakten Aufbau ein zuverlässiges und schonendes Abscheiden von Gasblasen aus einer Kardioplegielösung ermöglicht wird.

Die Aufgabe wird zum einen durch ein Verfahren mit den Merkmalen des Anspruchs 1 und zum anderen mit einem Blasenabscheider mit den Merkmalen des Anspruchs 4 gelöst. Bevorzugte Ausführungen der Erfindung sind in den jeweils abhängigen Ansprüchen angegeben.

Das erfindungsgemäße Verfahren ist dadurch gekennzeichnet, dass als Filterelement eine dünnwandige Hülse mit einem Filtermaterial verwendet wird, welches mit einer Poren- oder Maschenweite gebildet ist, welche kleiner als 50 µm ist. Ein Grundgedanke der Erfindung kann darin gesehen werden, dass als Filterelement ein Filtermaterial, insbesondere ein Filtergewebe verwendet wird, welches besonders fein ist und eine Poren- oder Maschenweite aufweist, welche kleiner als 50 µm ist. Damit können auch sehr feine Gasblasen in der Kardioplegielösung rückgehalten werden, was für den Patienten besonders schonend ist. Zudem wird durch die Ausbildung des Filterelementes als eine dünnwandige Hülse erreicht, dass aufgrund der geringen Wanddicke, welche vorzugsweise zwischen 15 µm und 80 µm liegt, die Kardioplegielösung ohne großen Druckverlust das Filterelement durchströmen kann.

Eine bevorzugte Ausführungsform des erfindungsgemäßen Verfahrens besteht darin, dass das Filtermaterial ein Filtergewebe aufweist, dessen Maschenweite kleiner als 50 µm ist. Bei einem Gewebe können grundsätzlich besonders genau definierte Maschenweiten eingestellt werden, wobei Maschenweiten von 50 µm und kleiner trotz der hohen Feinheit eine sehr geringe Wanddicke und damit einen sehr geringen Druckverlust ermöglichen.

Eine weitere erfindungsgemäße Verfahrensvariante liegt darin, dass die Kardioplegielösung vor oder nach dem Abscheiden der Gasblasen gekühlt wird. Vorzugsweise erfolgt eine Kühlung nach dem Abscheiden der Gasblasen, da durch die Kühlung die Größe der Gasblasen verändert, insbesondere verkleinert wird.

Der erfindungsgemäße Blasenabscheider ist dadurch gekennzeichnet, dass das Filterelement als eine dünnwandige Hülse ausgebildet ist, welche ein Filtergewebe aufweist, und dass das Filtergewebe mit einer Maschenweite gewebt ist, welche kleiner als 50 µm ist. Mit einem derartig feinen Filtergewebe mit einer gleichbleibend kleinen Maschenweite von unter 50 µm können auch sehr kleine Gasblasen in einer Flüssigkeit, insbesondere einer Kardioplegielösung, sicher zurückgehalten werden.

Nach einem weiteren Aspekt der Erfindung ist das Filterelement als eine einfache dünnwandige Hülse ausgebildet. Aufgrund dieser einfachen geometrischen Form mit einer sehr kleinen Wanddicke, welche vorzugsweise zwischen 15 µm und 80 µm liegt, kann die zu behandelnde Flüssigkeit ohne großen Druckverlust das Filterelement durchströmen. Vorzugsweise ist der Blasenabscheider ohne einen Wärmetauscher ausgebildet. Allerdings ist es für bestimmte Anwendungsfälle möglich, dass der erfindungsgemäße Blasenabscheider zusätzlichen mit einem Wärmetauscher versehen ist.

Eine bevorzugte Ausführungsform der Erfindung besteht darin, dass die Hülse zylindrisch oder konisch ausgebildet ist. Eine zylindrische oder konische Form sichert eine gute Stabilität auch bei einer dünnen Wandstärke des entsprechend geformten Filterelementes. Darüber hinaus werden über die gesamte Fläche weitgehend gleiche Einströmverhältnisse geschaffen. Das hülsenförmige Filterelement kann dabei einen Rahmenkörper aufweisen, auf welchem das Filtergewebe zum Bilden einer im Wesentlichen zylindrischen oder konischen Filtrationsfläche aufgespannt oder gehalten ist. Der Rahmenkörper weist vorzugsweise einen unteren und einen oberen Haltering auf, welche über schmale Stege miteinander verbunden sind.

Eine weitere Verbesserung bei der Blasenabscheidung wird nach einer weiteren Ausführungsvariante der Erfindung dadurch erreicht, dass sich die Hülse nach oben zu der oberen Auffangkammer hin im Durchmesser erweitert. Dabei ist eine Einströmung der Flüssigkeit in den Blasenabscheider in einem Mittenbereich der Hülse vorgesehen, wobei die Flüssigkeit radial nach außen strömt. Dabei scheiden sich größere Gasblasen an der radialen Innenseite des hülsenförmigen Filterelementes ab. Aufgrund der sich nach oben öffnenden Konizität können die einzelnen Blasen akkumulieren, sich vergrößern und dann nach oben in der Flüssigkeit zur Auffangkammer aufsteigen.

Für einen länger andauernden Betrieb sowie zur Sicherstellung gleichbleibender Druckverhältnisse in dem Blasenabscheider ist es nach einer Weiterbildung der Erfindung vorteilhaft, dass die Auffangkammer eine obere Ablassöffnung zum Austritt des abgeschiedenen Gases aufweist. Die Ablassöffnung kann durch ein Ventil verschließbar sein. Das Ventil kann zu bestimmten Zeitpunkten oder bei Erreichen eines bestimmten Druckes geöffnet werden.

Eine besonders bevorzugte Ausführungsform der Erfindung besteht darin, dass der Zulauf in einer mittigen Eintrittskammer mündet, dass die mittige Eintrittskammer von einer ringförmigen Austrittskammer umgeben ist, welche mit dem Ablauf verbunden ist und dass die Eintrittskammer von der Austrittskammer durch das als Hülse ausgebildete Filterelement getrennt ist. Das hülsenförmige Filterelement ist dabei konisch gestaltet, wobei ein Aufsteigen der zurückgehaltenen Gasblasen weitgehend behinderungsfrei ermöglicht wird.

Zur Sicherstellung einer besonders guten Gasabscheidung ist es nach einer Ausführungsvariante der Erfindung vorgesehen, dass das Gewebe eine Maschenweite zwischen 15 µm und 40 µm aufweist. Dabei ist das Gewebe mit einer sehr gleichmäßigen feinen Maschenweite ausgebildet, wobei vorzugsweise Abweichungen in der Maschenweite nicht mehr als 20%, vorzugsweise nicht mehr als 15% betragen. Hierdurch wird eine zuverlässige Gasblasenabscheidung von Gasblasen sichergestellt, welche einen Durchmesser von größer 15 µm aufweisen.

Zur Erreichung einer hohen Feinheit und einer gleichmäßigen Maschenweite des Filtergewebes ist es nach einer Ausbildung der Erfindung vorgesehen, dass das Filtergewebe aus einem Kunststoff-Monofilgarn gebildet ist. Das Garn ist aus einem geeigneten Kunststoff, insbesondere PET hergestellt. Vorzugsweise ist das Filtergewebe medizinisch gewaschen und bevorzugt biokompatibel.

Ein sehr gutes Verhältnis zwischen Stabilität und Durchsatzleistung wird gemäß einer Ausführungsvariante der Erfindung dadurch erreicht, dass das Monofilgarn einen Durchmesser zwischen 10 µm bis 25 µm aufweist. Eine zusätzliche Verbesserung bei der Blasenabscheidung wird gemäß einer Weiterbildung der Erfindung dadurch erreicht, dass das Monofilgarn und/oder das Filtergewebe hydrophil ausgebildet sind. Hierdurch wird eine gute Durchströmung der Flüssigkeit durch das Filterelement bei einer gleichzeitig guten Ablösung der Gasblasen erreicht.

Eine besonders gute Durchsatzleistung ergibt sich nach einer weiteren Ausführungsform der Erfindung dadurch, dass das Gewebe eine offene Fläche von mindestens 20% und eine Dicke zwischen 20 µm und 60 µm aufweist. Vorzugsweise weist das Gewebe eine offene Fläche von bis zu 30% der Gesamtfläche des Gewebes, besonders bevorzugt zwischen 20% und 25% auf. Die Gewebebindung ist vorzugsweise eine Köperbindung, insbesondere ein 3:3 Köper.

Die Erfindung wird nachfolgend anhand eines bevorzugten Ausführungsbeispieles weiter beschrieben, welches schematisch und in vergrößertem Maßstab in den Zeichnungen dargestellt ist. In den Zeichnungen zeigen:
- Fig. 1: eine erste schematische perspektivische Ansicht eines erfindungsgemäßen Blasenabscheiders von unten;
- Fig. 2: eine zweite schematische perspektivische Ansicht des Blasenabscheiders von Fig. 1 von oben;
- Fig. 3: eine schematische Querschnittsansicht des Blasenabscheiders; und
- Fig. 4: eine Liniendarstellung des Blasenabscheiders.

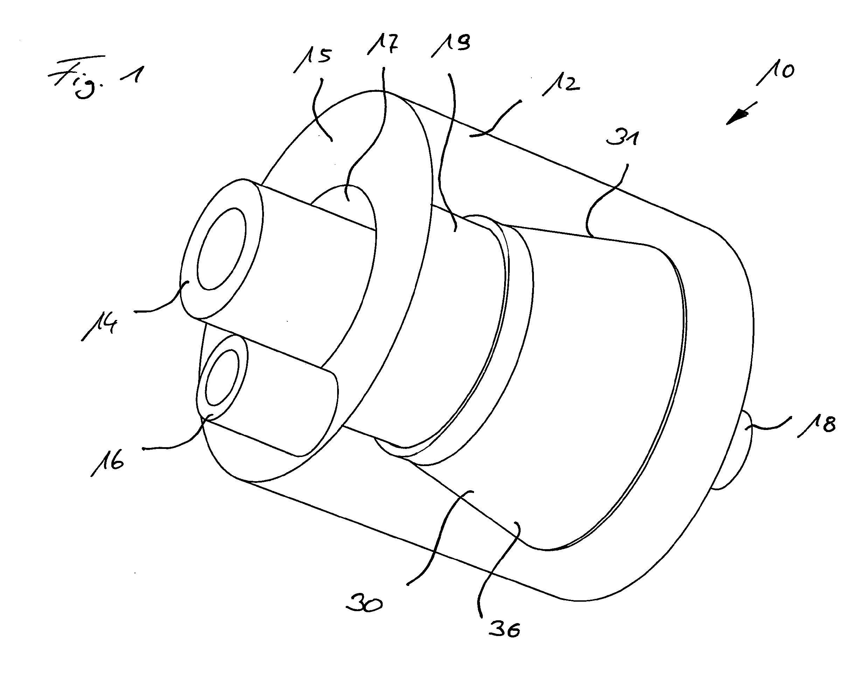

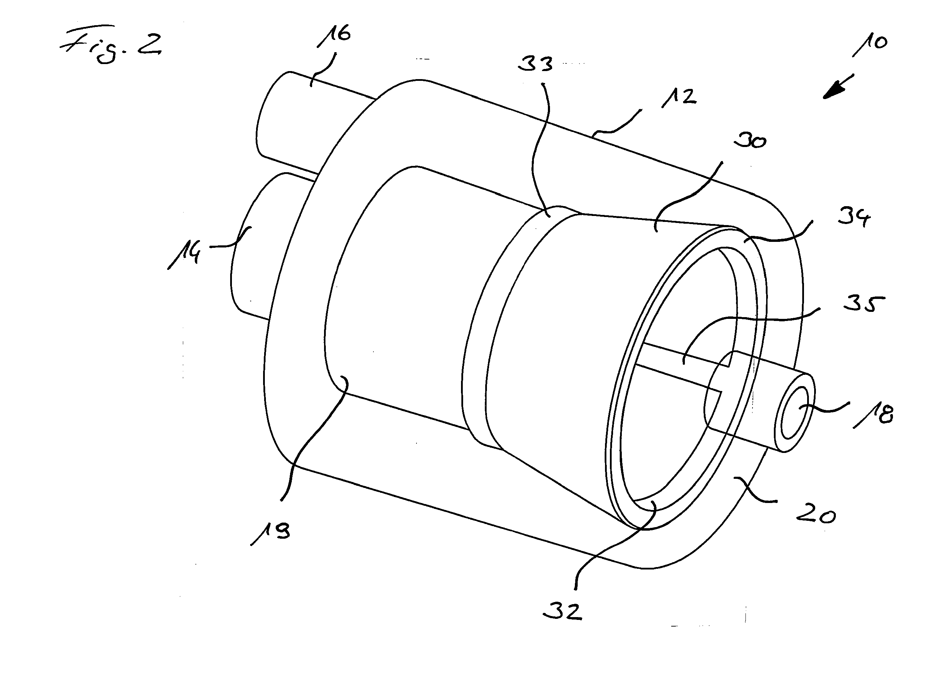

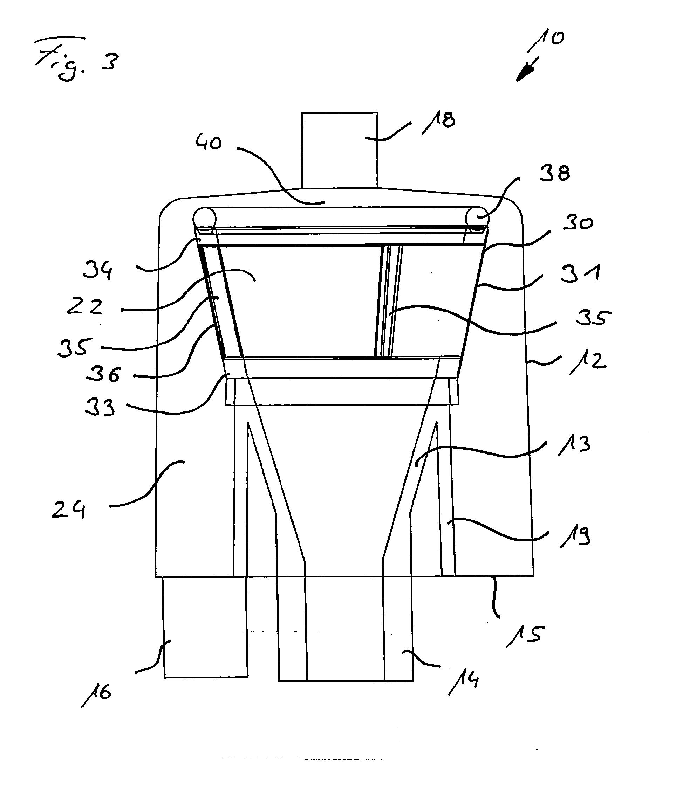

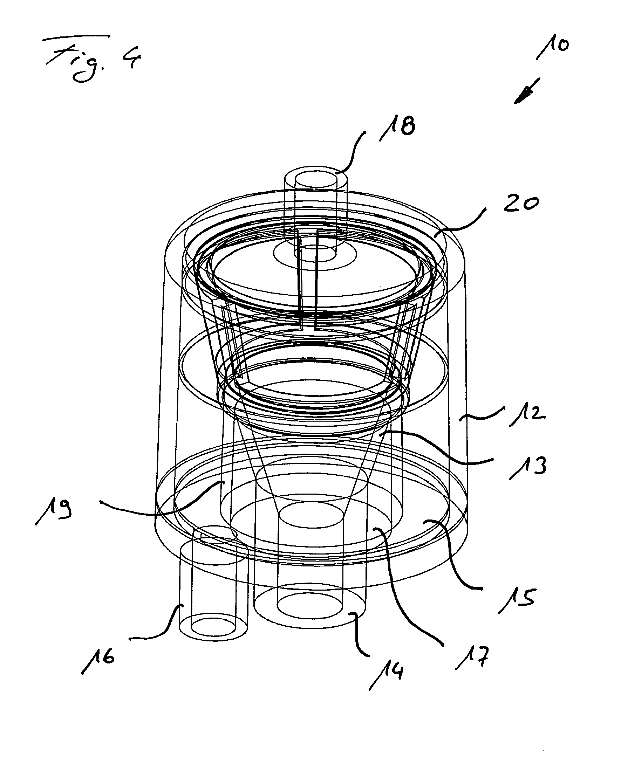

Ein erfindungsgemäßer Blasenabscheider 10 gemäß den Figuren 1 bis 4 umfasst ein etwa zylindrisches Gehäuse 12 an dessen unteren Bodenbereich ein stutzenartiger Zulauf 14 angeordnet ist. Radial versetzt zu dem mittigen Zulauf 14 ist ein stutzenartiger Ablauf 16 an dem Bodenbereich angebracht. Das Gehäuse 12 ist aus einem durchsichtigen Kunststoffmaterial gefertigt.

Der Bodenbereich weist eine Bodenplatte 15 mit einer Mittenöffnung 17 auf. Der rohrförmige Zulauf 14 erstreckt sich durch die Mittenöffnung 17 und geht in einen trichterförmigen Rohrabschnitt 13 über, auf welchem ein hülsenförmiges Filterelement 30 angeordnet ist. Der Rohrabschnitt 13 ist über eine zylindrische Außenwand 19 mit der Bodenplatte 15 und folglich mit dem Gehäuse 12 verbunden.

Innerhalb des Gehäuses 12 ist in einem oberen Bereich das Filterelement 30 angeordnet, welches als eine Hülse 31 ausgebildet ist. Das hülsenartige Filterelement 30 weist einen Rahmenkörper 32 mit einem durchmesserkleineren unteren Haltering 33 und einem durchmessergrößeren oberen Haltering 34 auf. Der untere Haltering 33 und der obere Haltering 34 sind über im Wesentlichen axial verlaufende Stege 35 zu dem einstückigen Rahmenkörper 32 verbunden. Der konische Rahmenkörper 32 ist aus einem Kunststoffmaterial gebildet, vorzugsweise spritzgegossen. Zum Bilden des hülsenförmigen Filterelementes 30 ist an dem Rahmenkörper 32 ein Filtergewebe 36 aufgespannt.

Das Filtergewebe 36 ist aus einem Kunststoff-Monofilgarn, vorzugsweise aus PET mit einer Köper-Bindung gewebt. Das Monofilamentgewebe ist hydrophil und/oder medizinisch ausgerüstet. Das Kettgarn des Filtergewebes 36 weist einen Durchmesser von 24 µm und das Schussgarn weist einen Durchmesser von 19 µm auf. Die Maschenweite beträgt 20 µm mit einer Abweichung von +/- 3 µm. Die Dicke des Filtergewebes 36 beträgt bei dem Ausführungsbeispiel 42 µm, wobei ein Gewicht von 24 g/m² erreicht wird. Bei den angeführten Gegebenheiten beträgt die Dicke des Filtergewebes 36 42 µm bei einer offenen Fläche von 22,5%.

Das obere Ende des Gehäuses 12 ist durch einen Deckel 20 gebildet. Das Filterelement 30 grenzt mit einer Ringdichtung 38 radial dicht an den Deckel 20 an. Im Mittenbereich des Deckels 20 ist eine stutzenartige Auslassöffnung 18 ausgebildet.

Der erfindungsgemäße Blasenfilter 10 wird in einer im Wesentlichen vertikalen Anordnung, insbesondere für eine medizinische Behandlung, in eine Zuführleitung für eine Kardioplegielösung geschaltet, welche zum Einleiten der Lösung in einen Patienten vorgesehen ist. Zum Abscheiden auch feiner Gasblasen in der Kardioplegielösung strömt diese von unten über den Zulauf 14 über den im Gehäuse 12 liegenden Rohrabschnitt 13 zum unteren Ende des hülsenförmigen Filterelement 30 und strömt durch den unteren Haltering 33 in eine Eintrittskammer 22 innerhalb des hülsenartigen Filterelementes 30. Von der mittigen Eintrittskammer 22 strömt die Lösung radial in allen Richtungen durch das feine Filtergewebe 36 des Filterelementes 30, wobei entsprechend der Maschenweite des Filtergewebes 36 durchmessergrößere Gasblasen an der Innenseite des Filtergewebes 36 rückgehalten werden.

Die filtrierte Kardioplegielösung gelangt in eine ringförmige Austrittskammer 24, welche das hülsenförmige Filterelement 30 ringförmig umgibt. Von der Austrittskammer 24 strömt die entgaste Flüssigkeit über den Ablauf 16 nach unten aus dem Gehäuse 12 und kann beispielsweise zu einem Patienten geleitet werden.

Im Laufe des Betriebs des Blasenabscheiders 10 sammeln sich in der Eintrittskammer 22 entlang der Innenseite des Filtergewebes 36 Gasblasen an, welche sich mit der Zeit akkumulieren und somit größere Gasblasen bilden. Bei einer üblicherweise vertikalen Anordnung des Blasenabscheiders 10 lösen sich die Gasblasen von dem Filtergewebe 36 und können in der Kardioplegielösung in der Eintrittskammer 22 des sich nach oben konisch erweiternden Filterelements 30 nach oben zu dem Deckel 20 strömen. Im Bereich des Deckels 20 ist eine Auffangkammer 40 für die Gasblasen vorgesehen. Über die Ablassöffnung 18, an welcher ein Auslassventil vorgesehen sein kann, kann so das ausfiltrierte Gas nach außen zur Atmosphäre abgeleitet werden.

## Patentansprüche

1. Verfahren zum Abscheiden von Gasblasen aus einer Kardioplegielösung mit einem Blasenabscheider (10), wobei
- die Kardioplegielösung über einen Zulauf (14) in ein Gehäuse (12) mit einem Filterelement (30) geleitet wird,
- die Kardioplegielösung das Filterelement (30) durchströmt, wobei Gasblasen in der Kardioplegielösung an dem Filterelement (30) rückgehalten werden, und
- die zurückgehaltenen Gasblasen an dem Filterelement (30) nach oben zu einer Auffangkammer (40) abgeführt werden,
**dadurch gekennzeichnet,**
**dass** als Filterelement (30) eine dünnwandige Hülse (31) mit einem Filtermaterial verwendet wird, welches mit einer Poren- oder Maschenweite gebildet ist, welche kleiner als 50 µm ist.

2. Verfahren nach Anspruch 1,
**dadurch gekennzeichnet,**
**dass** das Filtermaterial ein Filtergewebe (36) aufweist, dessen Maschenweite kleiner als 50 µm ist.

3. Verfahren nach Anspruch 11 oder 12,
**dadurch gekennzeichnet,**
**dass** die Kardioplegielösung vor oder nach dem Abscheiden der Gasblasen gekühlt wird.

4. Blasenabscheider zum Abscheiden von Gasblasen aus einer Flüssigkeit, insbesondere aus einer Kardioplegielösung nach einem Verfahren nach einem der Ansprüche 1 bis 3, mit
- einem Gehäuse (12), welches einen Zulauf (14) und einen Ablauf (16) für die Flüssigkeit aufweist,
- einem in dem Gehäuse (12) angeordneten Filterelement (30), welches von der Flüssigkeit durchströmbar ist, wobei Gasblasen in der Flüssigkeit an dem Filterelement (30) rückgehalten werden, und
- einer Auffangkammer (40), welche oberhalb des Filterelementes (30) in dem Gehäuse (12) angeordnet und zum Auffangen der zurückgehaltenen Gasblasen ausgebildet ist,
**dadurch gekennzeichnet,**
- dass das Filterelement (30) als eine dünnwandige Hülse (31) ausgebildet ist, welche ein Filtergewebe (36) aufweist, und
- dass das Filtergewebe (36) mit einer Maschenweite gewebt ist, welche kleiner als 50 µm ist.

5. Blasenabscheider nach Anspruch 5,
**dadurch gekennzeichnet,**
**dass** die Hülse (31) zylindrisch oder konisch ausgebildet ist.

6. Blasenabscheider nach Anspruch 4 oder 5,
**dadurch gekennzeichnet,**
**dass** sich die Hülse (31) nach oben zu der oberen Auffangkammer (40) hin im Durchmesser erweitert.

7. Blasenabscheider nach einem der Ansprüche 4 bis 6,
**dadurch gekennzeichnet,**
**dass** die Auffangkammer (40) eine obere Ablassöffnung (18) zum Austritt des abgeschiedenen Gases aufweist.

8. Blasenabscheider nach einem der Ansprüche 4 bis 7,
**dadurch gekennzeichnet,**
**dass** der Zulauf (14) in einer mittigen Eintrittskammer (22) mündet,
**dass** die mittige Eintrittskammer (22) von einer ringförmigen Austrittskammer (24) umgeben ist, welche mit dem Ablauf (16) verbunden ist, und
**dass** die Eintrittskammer (22) von der Austrittskammer (24) durch das als Hülse (31) ausgebildete Filterelement (30) getrennt ist.

9. Blasenabscheider nach einem der Ansprüche 4 bis 8,
**dadurch gekennzeichnet,**
**dass** das Filtergewebe (36) eine Maschenweite zwischen 15 µm und 50 µm aufweist.

10. Blasenabscheider nach einem der Ansprüche 4 bis 9,
**dadurch gekennzeichnet,**
**dass** das Filtergewebe (36) aus einem Kunststoff-Monofilgarn gebildet ist.

11. Blasenabscheider nach Anspruch 10,
**dadurch gekennzeichnet,**
**dass** das Monofilgarn einen Durchmesser zwischen 10 µm bis 25 µm aufweist.

12. Blasenabscheider nach Anspruch 10 oder 11,
**dadurch gekennzeichnet,**
**dass** das Monofilgarn und/oder das Filtergewebe (36) hydrophil ausgerüstet sind.

13. Blasenabscheider nach einem der Ansprüche 4 bis 12,
**dadurch gekennzeichnet,**
**dass** das Filtergewebe (36) eine offene Fläche von mindestens 20% und eine Dicke zwischen 20 µm und 60 µm aufweist.
